(19) **Europäisches Patentamt**

**European Patent Office**

**Office européen des brevets**

(11) **EP 0 969 849 B1**

(12) **EUROPÄISCHE PATENTSCHRIFT**

(45) Veröffentlichungstag und Bekanntmachung des Hinweises auf die Patenterteilung:
**23.07.2003   Patentblatt 2003/30**

(51) Int Cl.$^7$: **A61K 33/06**, A61K 31/59
// (A61K33/06, 31:59, A61P19:08, 19:10)

(21) Anmeldenummer: **98910722.2**

(22) Anmeldetag: **04.03.1998**

(86) Internationale Anmeldenummer:
**PCT/EP98/01208**

(87) Internationale Veröffentlichungsnummer:
**WO 98/040085 (17.09.1998 Gazette 1998/37)**

(54) **STABILE, FESTE ZUBEREITUNG ENTHALTEND VITAMIN D3 UND TRICALCIUMPHOSPHAT**

STABLE SOLID PREPARATION CONTAINING VITAMIN D3 AND TRICALCIUM PHOSPHATE

COMPOSITION SOLIDE STABLE CONTENANT DE LA VITAMINE D3 ET DU PHOSPHATE TRICALCIQUE

(84) Benannte Vertragsstaaten:
**AT BE CH DE DK ES FI FR GB GR IE IT LI LU NL PT SE**
Benannte Erstreckungsstaaten:
**LT LV RO SI**

(30) Priorität: **12.03.1997  DE 19710054**

(43) Veröffentlichungstag der Anmeldung:
**12.01.2000   Patentblatt 2000/02**

(73) Patentinhaber: **MERCK PATENT GmbH**
**64271 Darmstadt (DE)**

(72) Erfinder:
• **SCHÄFFLER, Achim**
**D-64743 Beerfelden (DE)**

• **WILDNER, Claudia**
**D-64625 Bensheim (DE)**

(56) Entgegenhaltungen:
**EP-A- 0 413 828          EP-A- 0 588 539**
**WO-A-96/09036**

• **CHAPUY ET AL.: "Vitamin D3 and calcium to prevent hip fructures in the elderly women" NEW ENGLAND J MEDICINE, Bd. 327, Nr. 23, 1992, Seiten 1637-1642, XP002069715**

Bemerkungen:
Die Akte enthält technische Angaben, die nach dem Eingang der Anmeldung eingereicht wurden und die nicht in dieser Patentschrift enthalten sind.

**Beschreibung**

Gegenstand der Erfindung ist eine pharmazeutische Zubereitung enthaltend eine Kombination aus einer Form von Vitamin $D_3$ und Tricalciumphosphat.

Diese neue Zubereitung hat eine verbesserte Stabilität und kann z.B. zur Prävention von Osteoporose, zur Behandlung von Vitamin $D_3$- und/oder Calcium-Mangelzuständen sowie zur Prävention der Pagetschen Krankheit verwendet werden.

Gegenstand der Erfindung ist weiterhin eine pharmazeutische Zubereitung, dadurch gekennzeichnet, daß 100 bis 1400 IU einer aktiven Form von Vitamin $D_3$ und 1000 bis 4500 mg Tricalciumphosphat eingesetzt werden. 1000 bis 4500 mg Tricalciumphosphat entsprechen 360 bis 1640 mg Calcium.

Bevorzugt werden 200 bis 1200 IU, insbesondere 400 bis 1000 IU einer aktiven Form von Vitamin $D_3$ eingesetzt. Weiterhin bevorzugt werden 1300 bis 4000 mg Tricalciumphosphat, insbesondere 1600 bis 3500 mg eingesetzt.

Gegenstand der Erfindung ist weiterhin eine pharmazeutische Zubereitung, dadurch gekennzeichnet, daß es sich um eine feste Zubereitung in Form von Pulver oder Granulaten handelt.

Ein weiterer Gegenstand der Erfindung ist eine pharmazeutische Zubereitung, dadurch gekennzeichnet, daß es sich um eine feste Zubereitung zur oralen täglichen Einmalapplikation, abgefüllt in Sachets oder Tabletten, handelt.

Gegenstand der Erfindung ist auch eine pharmazeutische Zubereitung, dadurch gekennzeichnet, daß es sich um eine Suspension handelt, die aus Pulvern vor der Einnahme hergestellt wird.

Der Erfindung lag die Aufgabe zugrunde, neue Arzneimittel in Form von pharmazeutischen Zubereitungen zur Verfügung zu stellen, die bessere Eigenschaften besitzen als bekannte, für die gleichen Zwecke verwendbare Arzneimittel.

Diese Aufgabe wurde durch das Auffinden der neuen Zubereitung gelöst. Überraschenderweise wurde gefunden, daß eine aktive Form von Vitamin $D_3$ zusammen mit Tricalciumphosphat zu einem Kombinationspräparat verarbeitet werden können.

Tricalciumphosphat hat die Formel $Ca_3(PO_4)_2$.

Als aktive Formen von Vitamin $D_3$ sind insbesondere solche aktiven Vitamin $D_3$-Formen bevorzugt, in denen die aktive Form eine Hydroxylgruppe in der 1α-Position trägt, z.B. 1α-Hydroxycholecalciferol, 1α, 25-Dihydroxycholecalciferol, 1α, 24-Dihydroxycholecalciferol, 1α, 24, 25-Trihydroxycholecalciferol, 1α-Hydroxy-24-oxocholecalciferol, 1α, 25-Dihydroxy-24-oxo-cholecalciferol, 1α, 25-Dihydroxy-cholecalciferol-26,23-lacton, 1α, 25-Dihydroxy-cholecalciferol-26,23-peroxylacton oder 26,26,26,27,27,27-Hexafluoro-1α, 25-dihydroxycholecalciferol. Ferner sind bevorzugt z.B. 25-Hydroxycholecalciferol, 24-Hydroxycholecalciferol, 24-Oxocholecalciferol, 24,25-Dihydroxycholecalciferol, 25-Hydroxy-24-oxo-cholecalciferol, 25-Hydroxycholecalciferol-26,23-lacton oder 25-Hydroxycholecalciferol-26,23-peroxylacton.

Ganz besonders bevorzugt ist 1α-Hydroxycholecalciferol. Dieses wird auch einfach Vitamin $D_3$ oder Cholecalciferol genannt

In der EP 0 413 828 und EP 0 702 954 sind bereits pharmazeutische Präparate genannt, die Kombinationen von Vitamin $D_3$ und Calciumsalzen enthalten.

In EP 0 413 828 wird eine pharmazeutische feste Zubereitung einer aktiven Form von Vitamin $D_3$ beansprucht, umfassend eine aktive Form von Vitamin $D_3$, die in einem organischen Lösungsmittel leicht löslichen Exzipienten dispergiert ist, und eine basische Substanz enthält.

Unter den basischen Substanzen wird Tricalciumphosphat nicht genannt.

Die basische Substanz dient zur Stabilisierung von Vitamin $D_3$.

In EP 0 702 954 wird eine pharmazeutische Zubereitung beschrieben, die ein Calciumsalz, Vitamin D und wenigstens eine Bor-, Kupfer- oder Magnesiumverbindung, enthält. In der zur Osteoporose-Behandlung geeigneten Zusammensetzung können 1000 bis 2500 mg Calciumsalz, entsprechend 400 bis 1000 mg Calcium, enthalten sein. Tricalciumphosphat ist als Calciumsalz nicht genannt.

Die genannten Bor- und Kupfer-Verbindungen könnten bei längerer Anwendung zu unerwünschten Nebenwirkungen führen.

Im japanischen Schutzrecht JP 05255095 wird eine Komposition beschrieben, die ein mikrokristallines Pulver von Calciumphosphat (Hydroxylapatit oder Calciumtriphosphat) und z.B. Vitamin $D_3$ enthält. Der Gegenstand zielt jedoch auf die i.v.-Applikation und die Anwendung an Blutgefäßen ab.

WO 96/09036 A1 offenbart Zusammensetzungen enthaltend Vitamin $D_3$ und Calciumcarbonat, wobei letzteres gegen Calciumtriphosphat austauschbar sein soll. Calciumtriphosphat weist die chemische Formel $Ca_5(P_3O_{10})_2$ auf und ist nicht identisch mit Tricalciumphosphat, das die chemische Formel $Ca_3(PO_4)_2$ aufweist.

Chapuy M.C. et al. 1992 lehrt die Verabreichung von Vitamin $D_3$ und Tricalciumphosphat in verschiedenen, voneinander getrennt vorliegenden Darreichungsformen, und zwar die Verabreichung von Vitamin $D_3$ als Pillen und die Verabreichung von Tricalciumphosphat als Suspension.

Als Calciumquelle sind für den Fachmann aus dem Stand der Technik zusätzlich Calciumgluconat, -lactat, -citrat, dibasisches Calciumphosphat oder vorzugsweise Calciumcarbonat bekannt.

Im Vergleich zum Stand der Technik weist die erfindungsgemäße Zubereitung eine überraschende Stabilität auf.

Die Daten der Stabilitätsuntersuchung ist in Tabelle I am Beispiel einer Charge angegeben.

In EP 0 413 828 sind Ergebnisse von Stabilitätstests auf Seite 5 in Tabelle I offengelegt.

Überraschenderweise kann, im Gegensatz zu EP 0 413 828, die erfindungsgemäße Zusammensetzung ohne die Verwendung organischer Lösungsmittel, die das Ziel haben klebende Hilfsstoffe zu lösen, herstellt werden.

Im Vergleich zu dem nach 30 Tagen bei 40 °C und 75 % rel. Feuchte gemessenen Vitamin $D_3$-Gehalt von 91 %, beschrieben in EP 0 413 828, Seite 5, weist die erfindungsgemäße Zusammensetzung unter den gleichen Meßbedingungen, allerdings nach 13 Wochen, noch einen Vitamin $D_3$-Gehalt von 97 % auf.

Tabelle I

| Stabilität der Charge 41/95; hergestellt analog Beispiel 2 | | | | | | |
|---|---|---|---|---|---|---|
| Chargen Nr. 41/95 | Aussehen, Geruch, Geschmack | Gehalt Cholecalciferol [I.U.] | Abbauprodukte Cholecalciferol | Wasser-gehalt [%] | Ca-gehalt [g] | pH-Wert |
| Start | entspricht | 918 | 0,5 % Tc. | 1,60 | 1,18 | 6,2 |
| 25°C/60 % rel. Feuchte | | | | | | |
| 13 Wochen | entspricht | 897 | 1 % Ts. | 1,64 | 1,19 | 6,2 |
| 26 Wochen | entspricht | 879 | 1,3 % Tc. 0,9 % Ts. | 1,77 | 1,19 | 6,3 |
| 52 Wochen | entspricht | 849 | 1,2 % Tc. 0,6 % Ts. | 1,45 | 1,19 | 6,2 |
| 30°C/60 % rel. Feuchte | | | | | | |
| 26 Wochen | entspricht | 861 | 1,5 % Tc. | 1,77 | 1,18 | 6,2 |
| | | | 0,6 % Ts. | | | |
| 52 Wochen | entspricht | 826 | 1,3 % Tc. 0,6 % Ts. | 1,41 | 1,19 | 6,2 |
| 40°C/75 % rel. Feuchte | | | | | | |
| 13 Wochen | entspricht | 890 | u.N. | 1,72 | 1,19 | 6,1 |
| 26 Wochen | entspricht | 757 | 0,6 % Tc. | 1,82 | 1,19 | 6,2 |
| u.N.: unter Nachweisgrenze Tc.: Transcholecalciferol Ts.: Tachysterol I.U.: internationale Einheit | | | | | | |

Gegenstand der Erfindung ist auch ein Verfahren zur Herstellung einer pharmazeutischen Zubereitung, dadurch gekennzeichnet, daß man eine aktive Form von Vitamin $D_3$ und Tricalciumphosphat zusammen mit mindestens einem festen Träger- oder Hilfsstoff durch Mischen in eine geeignete Dosierungsform bringt.

Bevorzugt wird die aktive Form von Vitamin $D_3$ in Form eines festen Konzentrates eingesetzt. Als Cholecalciferol-Konzentrate sind z.B.

Duphasol D3-1000® dry stable oder Vitamin D3 Typ 100 CWS® bevorzugt.

Als Träger- und Hilfsstoffe (Bindemittel und/oder Viskositätserhöher) kommen in Frage z.B. Mannitol, Hydroxypropylcellulose, Lactose, Polyvinylpyrrolidon, Polyvinylalkohol, Gelatine, Stärke, kristalline Cellulose, Hydroxypropylmethylcellulose, Ethylcellulose, Carboxymethylcellulose, Dextrin, Lactose, Sorbitol, Sucrose, Talk (Magnesiumsilicat Hydrat), Kaolin, gefälltes Calciumcarbonat, Natriumchlorid, Titanoxid, Gummi Arabicum undloder Xanthan Gummi.

Als Fließregulierungsmittel eignet sich z.B. Aerosil.

Weitere Träger- oder Hilfsstoffe können zugesetzt werden, wie z.B. Bindemittel, Antioxidantien, Farbstoffe, Gleitmittel, Süßungsmittel und/oder Aromastoffe.

Bevorzugte Antioxidantien sind z.B. Butylhydroxytoluol (BHT), Propylgallat, Butylhydroxyanisol (BHA), Lecithin, $\alpha$-Tocopherol, Hydrochinon, Octylgallat, Dodecylgallat, Isoamylgallat, Nordihydroguaialansäure, Guaianharz, $\alpha$-Naphthylamin, Ethylprotocathecuat, Ascorbinsäure-stearinsäure-ester, Ascorbinsäurepalmitat, Cysteinhydrochlorid, Natriumsalz des Ascorbinsäurestearats, Thioglycerol oder Thiosorbitol.

Als Gleit- oder Schmiermittel sind bevorzugt z.B. Talk, Stärke, Magnesium- und Calciumstearat, Borsäure, Paraffin, Kakaobutter, Macrogol, Leucin oder Natriumbenzoat.

Als Süßungsmittel sind bevorzugt z.B. Aspartam oder Saccharin-Natrium, als Aromastoffe sind z.B. Zitronen- oder Orangenaroma bevorzugt.

Wie in Beispiel 1 angeführt, ist der Herstellungsgang dadurch gekennzeichnet, daß er, trotz hoher Anforderungen an die Gleichmäßigkeit der Verteilung des Wirkstoffes (siehe Beispiel 2; 20 µg Cholecaiciferol in 4,1 g Pulver pro Sachet) durch einfaches Mischen, und ohne Sprühgranulation, die kostenaufwendig ist und sich stabilitätsbeeinträchtigend auswirkt, auskommt.

Die neue pharmazeutische Zubereitung kann hergestellt werden, indem man eine aktive Form von Vitamin $D_3$ und Tricalciumphosphat, zusammen mit mindestens einem festen Träger- oder Hilfsstoff in eine geeignete Dosierungsform bringt. Die so erhaltenen Zubereitungen können als Arzneimittel in der Human- oder Veterinärmedizin, insbesondere bei der Prävention von Osteoporose und Oberschenkelhalsfrakturen sowie bei der Behandlung von Vitamin $D_3$- und/oder Calcium-Mangelzuständen sowie zur Prävention der Pagetschen Krankheit, eingesetzt werden. Als Trägersubstanzen kommen organische oder anorganische Stoffe in Frage, die sich für die orale Applikation eignen und mit den Verbindungen nicht reagieren, beispielsweise Gelatine, Sojalecithin, Kohlehydrate wie Lactose, Mannitol oder Stärke, Magnesiumstearat, Talk, Cellulose. Zur oralen Anwendung dienen insbesondere Tabletten, Dragees, Kapseln, Suspensionen oder Granulate bzw. Pulver, abgefüllt in Sachets zur Verwendung in Suspensionen.

Die Zubereitungen können Hilfsstoffe wie Konservierungs-, Stabilisierungsund/oder Netzmittel, Emulgatoren, Farb- und/oder Aromastoffe enthalten.

Die tägliche einmalige Dosierung von Vitamin-$D_3$ und Tricalciumphosphat liegt vorzugsweise zwischen etwa 200 bis 1200 IU Vitamin $D_3$ und 1200 bis 4000 mg Tricalciumphosphat, insbesondere zwischen etwa 400 bis 1000 IU Vitamin $D_3$ und 1600 bis 3500 mg Tricalciumphosphat, ganz besonders zwischen etwa 700 bis 900 IU Vitamin $D_3$ und 3200 bis 3500 mg Tricalciumphosphat. Die orale Applikation ist bevorzugt.

Ganz bevorzugte Dosierungen enthalten 400 IU Vitamin $D_3$ und 1650 mg Tricalciumphosphat, bzw. die jeweils doppelte Menge, also 800 IU Vitamin $D_3$ und 3300 mg Tricalciumphosphat.

Die nachfolgenden Beispiele betreffen die Herstellung und die Zusammensetzung der erfindungsgemäßen pharmazeutischen Zubereitung

Beispiel 1:

Cholecalciferol-Konzentrat und Sorbit werden gesiebt und in einem Trommelmischer 30 Minuten lang miteinander gemischt (Mischung a).

Tricalciumphosphat, kolloidales wasserfreies Silica, Hydroxypropylmethylcellulose, Gummi Arabicum, Gelatine, Aspartam und Zitronenaroma werden gesiebt. Anschließend werden die Komponenten zusammen mit Mischung a in einem Trommelmixer 30 Minuten miteinander vermischt.

Die Mischung wird noch einmal gesiebt und anschließend noch einmal gemischt.

Alternativ kann das Mischen aller Komponenten auch in einem Schnellmischer durchgeführt werden.

Beispiel 2:

Cholecalciferol-Konzentrat und Lactose werden gesiebt und in einem Trommelmischer 30 Minuten lang miteinander gemischt (Mischung a).

Tricalciumphosphat, kolloidales wasserfreies Silica, Hydroxypropylmethylcellulose, Gummi Arabicum, Gelatine, Aspartam und Orangenaroma werden gesiebt. Anschließend werden die Komponenten zusammen mit Mischung a In einem Trommelmixer 30 Minuten miteinander vermischt.

Die Mischung wird noch einmal gesiebt und anschließend noch einmal gemischt.

Alternativ kann das Mischen aller Komponenten auch in einem Schnellmischer durchgeführt werden.

Beispiel 3

Zusammensetzung eines Pulvers, abgefüllt in ein Sachet, für eine orale Suspension, das 1,2 g Calcium und 20 µg Vitamin $D_3$ enthält:

| | |
|---|---|
| Cholecalciferol-Konzentrat* | 8,0 mg |
| Sorbit | 227 mg |

\* entspricht 800 IU Choelcalciferol

(fortgesetzt)

| Tricalciumphosphat** | 3300,0 mg |
|---|---|
| Kolloidales wasserfreies Silica | 130,0 mg |
| Hydroxypropylmethylcellulose | 150,0 mg |
| Gummi Arabicum | 175,0 mg |
| Saccharin Natriumsalz | 10,0 mg |
| natürliches Zitronenaroma | 100.0 mg |
| | 4100,0 mg |

** entspricht 1,2 g Calcium

Beispiel 4

Zusammensetzung eines Pulvers, abgefüllt in ein Sachet, für eine orale Suspension, das 0,6 g Calcium und 10 $\mu$g Vitamin $D_3$ enthält:

| Cholecalciferol-Konzentrat* | 4,0 mg |
|---|---|
| Sorbit | 114,0 mg |
| Tricalciumphosphat** | 1650,0 mg |
| Kolloidales wasserfreies Silica | 65,0 mg |
| Hydroxypropylmethylcellulose | 75,0 mg |
| Gummi Xanthan | 87,0 mg |
| Saccharin Natriumsalz | 5,0 mg |
| natürliches Zitronenaroma | 50,0 mg |
| | 2050, 0 mg |

* entspricht 400 IU Choelcalciferol

** entspricht 0,6 g Calcium

Beispiel 5

Zusammensetzung einer Suspension, die 1,2 g Calcium und 20 $\mu$g Vitamin $D_3$ enthält:

| Cholecalciferol-Konzentrat* | 8,0mg |
|---|---|
| Sorbit | 227 mg |
| Tricalciumphosphat** | 3300,0mg |
| Kolloidales wasserfreies Silica | 130,0mg |
| Hydroxypropylmethylcellulose | 150,0mg |
| Gummi Arabicum | 175,0mg |
| Saccharin Natriumsalz | 10,0mg |
| natürliches Zitronenaroma | 100,0mg |
| gereinigtes Wasser | 10 ml |

* entspricht 800 IU Choelcalciferol

** entspricht 1,2 g Calcium

**Patentansprüche**

1. Pharmazeutische Zubereitung enthaltend eine Kombination aus einer aktiven Form von Vitamin $D_3$ und Tricalciumphosphat als Wirkstoffe.

2. Pharmazeutische Zubereitung nach Anspruch 1, **dadurch gekennzeichnet, daß** 100 bis 1400 IU einer aktiven Form von Vitamin $D_3$ und 1000 bis 4500 mg Tricalciumphosphat eingesetzt werden.

3. Pharmazeutische Zubereitung nach Anspruch 1 oder 2, **dadurch gekennzeichnet, daß** es sich um eine feste Zubereitung in Form von Pulver, Tabletten oder Granulaten handelt.

**4.** Pharmazeutische Zubereitung nach Anspruch 1, 2 oder 3, **dadurch gekennzeichnet, daß** es sich um eine feste Zubereitung zur oralen täglichen Einmalapplikation, abgefüllt in Sachets oder Tabletten, handelt.

**5.** Pharmazeutische Zubereitung nach Anspruch 1 oder 2, **dadurch gekennzeichnet, daß** es sich um eine Suspension handelt, die aus Pulvern vor der Einnahme hergestellt werden.

**6.** Verfahren zur Herstellung einer pharmazeutischen Zubereitung, **dadurch gekennzeichnet, daß** man eine aktive Form von Vitamin $D_3$ und Tricalciumphosphat zusammen mit mindestens einem festen Träger- oder Hilfsstoff durch Mischen in eine geeignete Dosierungsform bringt.

**7.** Verwendung einer aktiven Form von Vitamin $D_3$ sowie von Tricalciumphosphat zur Herstellung eines Arzneimittels zur Prävention von Osteoporose, zur Behandlung von Vitamin $D_3$- und/oder Calcium-Mangelzuständen sowie zur Prävention der Pagetschen Krankheit.

**Claims**

**1.** Pharmaceutical preparation comprising a combination of an active form of vitamin $D_3$ and tricalcium phosphate as active ingredients.

**2.** Pharmaceutical preparation according to Claim 1, **characterised in that** from 100 to 1400 IU of an active form of vitamin $D_3$ and from 1000 to 4500 mg of tricalcium phosphate are employed.

**3.** Pharmaceutical preparation according to Claim 1 or 2, **characterised in that** it is a solid preparation in the form of powders, tablets or granules.

**4.** Pharmaceutical preparation according to Claim 1, 2 or 3, **characterised in that** it is a solid preparation for oral administration once daily, delivered in sachets or tablets.

**5.** Pharmaceutical preparation according to Claim 1 or 2, **characterised in that** it is a suspension prepared from powders before being taken.

**6.** Process for the preparation of a pharmaceutical preparation, **characterised in that** an active form of vitamin $D_3$ and tricalcium phosphate together with at least one solid excipient or adjuvant are converted into a suitable dosage form by mixing.

**7.** Use of an active form of vitamin $D_3$ and of tricalcium phosphate for the preparation of a medicament for the prevention of osteoporosis, for the treatment of vitamin $D_3$ and/or calcium deficiency states and for the prevention of Paget's disease.

**Revendications**

**1.** Préparation pharmaceutique comprenant une association d'une forme active de la vitamine $D_3$ et de phosphate tricalcique comme ingrédients actifs.

**2.** Préparation pharmaceutique selon la revendication 1, **caractérisée en ce que** de 100 à 1 400 UI d'une forme active de la vitamine $D_3$ et de 1 000 à 4 500 mg de phosphate tricalcique sont employés.

**3.** Préparation pharmaceutique selon la revendication 1 ou 2, **caractérisée en ce qu'**il s'agit d'une préparation solide sous forme de poudres, de comprimés ou de granulés.

**4.** Préparation pharmaceutique selon la revendication 1, 2 ou 3, **caractérisée en ce qu'**il s'agit d'une préparation solide pour une administration orale une fois par jour, fournie en sachets ou en comprimés.

**5.** Préparation pharmaceutique selon la revendication 1 ou 2, **caractérisée en ce qu'**il s'agit d'une suspension préparée à partir de poudres avant d'être prise.

**6.** Procédé de préparation d'une préparation pharmaceutique, **caractérisé en ce qu'**une forme active de la vitamine $D_3$ et le phosphate tricalcique conjointement avec au moins un excipient ou adjuvant solide sont mis sous une forme de dosage convenable par mélange.

**7.** Utilisation d'une forme active de la vitamine $D_3$ et de phosphate tricalcique pour la préparation d'un médicament pour la prévention de l'ostéoporose, pour le traitement des états de déficience en vitamine $D_3$ et/ou en calcium et pour la prévention de la maladie de Paget.